# EUROPEAN PATENT SPECIFICATION

(11) **EP 1 689 489 B1**
(45) Date of publication and mention of the grant of the patent: **27.02.2008**
(21) Application number: 04811838.4
(22) Date of filing: 19.11.2004
(51) Int. Cl.: A61N 1/30

(54) **PEPTIDICALLY BUFFERED FORMULATIONS FOR ELECTROTRANSPORT APPLICATIONS AND METHODS OF MAKING**
PEPTIDISCH GEPUFFERTE FORMULIERUNGEN FÜR ELEKTROTRANSPORT-ANWENDUNGEN UND ZUGEHÖRIGE HERSTELLUNGSVERFAHREN
PREPARATIONS A TAMPON PEPTIDIQUE POUR DES APPLICATIONS PAR ELECTROTRANSPORT ET PROCEDES DE FABRICATION ASSOCIES

(30) Priority: 19.11.2003 US 523470 P
(43) Date of publication of application: 16.08.2006
(73) Proprietor: ALZA CORPORATION, Mountain View, CA 94039-7210 (US)
(72) Inventor: RAUSER, David, Gilroy, CA 95020 (US); PADMANABHAN, Rama, Los Altos, CA 94024 (US); PHIPPS, Joseph, B., Sunnyvale, CA 94087 (US); CORMIER, Michel, Mountain View, CA 94043 (US); SUBRAMONY, Janardhanan, Anand, Santa Clara, California 95051 (US)
(74) Representative: Williams, Paul Edwin
(86) International application number: PCT/US2004/039184
(87) International publication number: WO 2005/051485

(56) References cited:
- WO-A-96/34597
- WO-A-02/089803
- US-A- 6 071 508
- US-A1- 2002 058 608

## Description

### FIELD OF THE INVENTION

The present invention relates to drug formulations for delivery by electrotransport, electrotransport systems, and methods for preparing such drug formulations that involve adjusting the pH of a drug formulation to render it suitable for incorporation into an electrotransport delivery system.

### BACKGROUND OF THE INVENTION

The delivery of active agents through the skin provides many advantages, including comfort, convenience, and non-invasiveness. In addition, gastrointestinal irritation and the variable rates of absorption and metabolism encountered in oral delivery are avoided. Transdermal delivery also provides a high degree of control over blood concentrations of any particular active agent.

Many active agents are not suitable for passive transdennal delivery because of their size, ionic charge characteristics, and hydrophilicity. One method for transdermal delivery of such active agents involves the use of electrical current to actively transport the active agent into the body through intact skin, which is known as electrotransport or iontophoretic drug delivery. In present electrotransport devices, at least two electrodes are used, which are disposed so as to be in intimate electrical contact with some portion of the skin. One electrode, called the active or donor electrode, is the electrode from which the active agent is delivered into the body. The other electrode, called the counter or return electrode, serves to close the electrical circuit through the body. In conjunction with the patient's skin, the circuit is completed by connection of the electrodes to a source of electrical energy, and usually to circuitry capable of controlling the current passing through the device. If the ionic substance to be driven into the body is positively charged, then the positive electrode (the anode) will be the active electrode and the negative electrode (the cathode) will serve as the counter electrode. If the ionic substance to be delivered is negatively charged, then the cathodic electrode will be the active electrode and the anodic electrode will be the counter electrode.

Electrotransport devices also require a reservoir or source of the active agent that is to be delivered or introduced into the body. Such reservoirs are connected to the anode or the cathode of the electrotransport device to provide a fixed or renewable source of one or more desired active agents. As electrical current flows through an electrotransport device, oxidation of a chemical species takes place at the anode while reduction of a chemical species takes place at the cathode. Both of these reactions generate a mobile ionic species with a charge state like that of the active agent in its ionic form. Such mobile ionic species are referred to as competitive species or competitive ions because the species compete with the active agent for delivery by electrotransport.

Many active agents exist in both free acid/base form and salt form. Although the salt forms of active agents are likely to have higher water solubility, the pH of an aqueous solution of the active agent salt may not be optimal from the standpoint of transdermal flux and stability of the drug at a particular pH. For example, human skin exhibits a degree of permselectivity to charged ions that is dependant upon the pH of the donor solution of an electrotransport device. Generally, for anodic donor reservoir solutions, transdermal electrotransport flux of a cationic species is optimized when the pH of the donor solution is about 4 to about 10, more preferably about 5 to about 8, and most preferably about 6 to about 7. Generally, for cathodic donor reservoir solutions, transdermal electrotransport flux of an anionic species is optimized when the pH of the donor solution is about 2 to about 6, and more preferably about 3 to about 5.

A problem that arises with the addition of pH-altering species (e.g., an acid or a base) to the active agent solution in an electrotransport device is that extraneous ions having the same charge as the active agent are introduced into the solution. These ions generally compete with the active agent ions for electrotransport through the body surface. For example, the addition of sodium hydroxide to raise the pH of a cationic active agent-containing solution will introduce sodium ions into the solution that will compete with the cationic active agent for delivery by electrotransport into the patient, thereby making the electrotransport delivery less efficient, i.e., less active agent will be delivered per unit of electrical current applied by the device.

To address this problem, methods have been developed for adjusting the pH of an active agent formulation prior to incorporation into an electrotransport delivery system that do not involve the introduction of extraneous ions. Such methods are described in U.S. Patent Nos. 6,071,508; 5,853,383; PCT Application Publication No. WO 96/34597; and U.S. Patent Application Publication No. 20020058608, which are incorporated by reference in their entireties.

Certain drugs such as benzamidine derivatives (e.g., ROH-4746) exhibit poor oral absorption and bioavailability. Iontophoresis has been shown to deliver the required therapeutic amount (20-40 mg) of ROH-4746 in a controlled fashion over a 24 hour period. The controlled delivery profiles are important since overdosing will lead to excessive bleeding and underdosing will lead to thrombosis. The drug also contains a number of pKa's, one acidic (2.6) and two basic (9.4, 11.6). In aqueous solutions containing the drug, the pH was found to be extremely low (<1) due to the low value of the drug's first pKa of 2.6. As a result, for ROH-4746, as well as many other drugs, a means of providing a desirable pH to the acceptable formulation is essential.

### SUMMARY OF THE INVENTION

In certain drugs, chemical stability is pH sensitive and the pH of active agents can shift during long-term storage. There is a need for methods and formulations that provide buffering capacity and reduce changes in the pH of active agents that occur during electrotransport and long-term storage. For example, aqueous stability studies conducted with the benzamidine derivative factor Xa inhibitor (e.g., ROH-4746) indicated that the drug was very susceptible to base catalysis. At a pH of 7.5, appreciable degradation was observed as early as three weeks at 25° C. Although aqueous stability was acceptable at low pH (pH < 4), the charge on the drug was not optimal and electrotransport was reduced. It was therefore important to maintain the pH level between 4 and 6 in an electrotransport composition.

In this invention, it has been discovered that certain drugs, such as benzamidine derivatives (e.g., ROH-4746), can be pH-adjusted with an ion exchange resin and that such a drug solution has better flux during electrotransport than the same drug pH-adjusted with inorganic agents (such as NaOH for benzamidine derivatives). It has also been found that certain ion exchanged drug solutions, such as benzamidine derivatives (e.g., ROH-4746), have pH shift during storage or during electrotransport that can be advantageously buffered with multipeptide(s). The present invention, in one aspect, provides a method to provide a drug composition with stable pH by using ion exchange to adjust the pH of a drug followed by buffering with multipeptides. In another aspect, the invention provides a drug composition that is stable in storage and in application in transdermal delivery by electrotransport. In one aspect, compositions for use in an electrotransport delivery system can be obtained by providing a drug solution with drug ions and associated counterions; adjusting the pH of the drug solution by contacting the drug solution first with an ion exchange material; separating the ion exchange material from the pH-adjusted drug solution; and incorporating a peptidic buffer in the pH-adjusted drug solution to maintain pH over time.

In one aspect, a method for making a benzamidine derivative formulation incorporating a multipeptide buffer for the delivery of ROH-4746, a Factor Xa inhibitor, by electrotransport, such as iontophoresis, is described. The multipeptide helps maintain formulation pH and avoids unwanted pH shifts that may affect the stability of the drug while maintaining its +1 charge during storage of the device as well as when in use.

In one aspect, the present invention provides a benzamidine derivative (BD) composition for use in an electrotransport delivery system. The composition contains a drug solution that has drug ions and associated counterions of BD. The composition is at a pH from 4.5 to 6.0 and contains a multipeptide buffer having an isoelectric point (pI) that falls on a steep slope of a titration curve of BD. The steep slope is proximate to and higher than a first pKa of BD.

Multipeptides have been shown to be extremely effective as a buffering medium for pH-adjusted drug solutions, especially drug solutions previously pH-adjusted with ion exchange. When used with iontophoresis, the peptidic buffers of the present invention work extremely well since they are immobile in an electrical field when used at the isoelectric point (pI).

The use of peptidic buffer for buffering pH-adjusted drug solution affords many advantages. Of course, peptidic buffer can be used to buffer drug solutions that have been pH-adjusted with bases such as NaOH, KOH, NH₄OH, etc. However, avoiding or minimizing the use of such bases, the combination of pH-adjusting the drug solution first with ion exchange and subsequently buffering with peptidic buffer minimizes competing ions while providing pH and chemical stability to the drug. Although solid ion exchange resin(s) can be used as buffering agent to aid in maintaining pH of the formulation, there is no need for solid ion exchange resins to be present. In fact, it is preferred that ion exchange resin be absent when buffering is done with multipeptide buffers. If a formulation is buffered (i.e., maintained at a certain pH) with the use of ion exchange resin in the formulation, typically after adding the resin to the drug, acid or base still needs to be added to fine tune the pH to the desired range. As a result, a competing ion, such as sodium ion from adding sodium hydroxide, for iontophoresis would be introduced into the formulation. As a further advantage of using peptidic buffer after pH adjustment, no solid material (such as ion exchange resins) is used for buffering, the formulation can therefore be easily mixed to achieve the desired pH and homogeneity, thereby facilitating ease of manufacture of the electrotransport systems.

With the selection of a multipeptide buffer having a pI at the desired pH range for storage or application of the drug, it is relatively easy to achieve the desired pH. As long as the pH of the drug solution after pH adjustment by ion exchange falls on the steep slope of the titration curve about an inflection point of the drug, the addition of a relatively small amount of multipeptide buffer having the right pI will readily adjust the pH to the desired point or range. Because the drug solution (before buffering) has been pH-adjusted with ion exchange to be at or near the pI of the multipeptide used for buffering, the addition of only a small amount of buffering multipeptide will shift and maintain pH of the drug solution at the desired pH near the pI of the multipeptide. This renders the buffering process simpler than buffering with ion exchange resins.

Further, the pH-adjusted drug solution, having been ion-exchanged prior to buffering, already has very little or no competing ions (e.g., strong base cations such as inorganic alkali cations, e.g., Na⁺ ion, K⁺ ions, NH₄⁺ ions, etc. in the case of ROH-4746). Thus, the present use of multipeptide for buffering will provide the significant advantage of keeping the competing ions to a very low concentration, preferably to a minimum, thereby improving drug delivery by electrotransport over time, either in storage or in use on an individual. Reducing the amount of multipeptides present and yet maintaining the pH at the desired range also will help to achieve a larger flux than otherwise. Additionally, multipeptide buffers exhibit excellent biocompatibility. Thus, tendencies for skin irritation are reduced when transdermal devices with the multipeptide buffers are used. Using peptidic buffers further reduces or avoids the use of ion exchange resin in the matrix of the transdermal device and provides the formulation with great biocompatibility and little or no skin irritation.

In some cases, if desired, after the drug solution has been adjusted to desired pH with a first ion exchange material and the first ion exchanged material has been removed, a second ion exchange material can be used to contact pH-adjusted drug solution to help maintain pH over time, in addition to using a peptidic buffer.

In certain embodiments of the invention, the drug ions are cationic, the associated counterions are anionic, the first ion exchange material is a polymeric anion exchange material, and the second ion exchange material, if used, is a polymeric anion or cation exchange material. In certain other embodiments of the invention, the drug ions are anionic, the associated counterions are cationic, the first ion exchange material is a polymeric cation exchange material, and the second ion exchange material, if used, is a polymeric cation or anion exchange material.

This invention is especially useful for maintaining the pH of a drug with at least one pKa below and at least one pKa above the pH of storage and application. Without a good buffering system, as the drug ions are transported to the individual when the transdermal electrotransport device is in use, the pH may shift. For a drug having at least one pKa below and at least one pKa above the pH of storage or application, if the pH shifts to area approaching any of the pKa that reduces ionization of the drug, the flux of drug will suffer. Thus, the present buffering system not only improves the storage of the drug in a drug composition, but also ensures adequate ionization to maintain flux in application.

### BRIEF DESCRIPTION OF THE DRAWINGS

FIG. 1 depicts the chemical structure of ROH-4746, a 2-[3-[4-(4-piperidinyloxy)anilino]-1propenyl]benzamidine derivative.

FIG. 2 shows how the increase in buffer concentration in the formulation affects the steady state ROH-4746 flux and pH shift during iontophoretic use, in dipeptide (His-Glu) buffered anode hydrogels.

FIG. 3 is a graph that shows the shift in pH of a drug solution of ROH-4746, over a 12-week period in varied storage conditions, in His-Glu (25 mM) buffered hydrogels containing 2.37% ROH 4746.

FIG. 4 is a graph that shows the storage stability of His-Glu (25 mM) buffered hydrogels containing 2.37% ROH 4746, over a 12-week period in varied storage conditions.

### DETAILED DESCRIPTION OF ILLUSTRATIVE EMBODIMENTS

This invention provides methods for making formulations using multipeptide buffers that will minimize undesirable pH shifts during storage and when applied to an individual with the use of ion exchange to adjust pH before peptidic buffering, to result in drug compositions with low competing ions for electrotransport drug delivery. Adjusting the pH of a drug with ion exchange avoids the introduction of competing ions. Subsequent buffering with a multipeptide buffer to maintain the pH of the resulting drug solution over time further minimizes or avoids the introduction of competing ions under electrotransport, such as iontophoresis. As used herein, the terms "adjusting," "adjust," and all variations thereof refer to changing by any measurable degree the pH of a solution or substance.

As used herein, the terms "contacting," "contact," and all variations thereof, refer to any means that directly or indirectly cause placement together of moieties, such that the moieties come into physical contact with each other. Contacting thus includes physical acts such as placing the moieties together in a container, combining the moieties, or mixing the moieties.

As used herein, the terms "drug" and "pharmaceutically active agent" refer to any chemical material or compound that induces a desired local or systemic effect, and can be delivered by electrotransport. The terms "drug ion" and "associated counterions" refer to either positively or negatively charged forms of a drug with which counterions of a charge opposite to that of the drug are associated.

Ionic drugs that can be used in the method of the present invention include drugs that have a stable pH that is near to or at a steep slope of the titration curve of the drug and where the pH of the pure drug in aqueous solution is outside of the pH range for efficient transdermal drug delivery and acceptable irritability, which generally is in the pH 3-7 range, for some drugs in the pH 4-7 range. The pH of the drug for such consideration can be the pH of a substantially pure solution of the drug at which the concentration of the drug is in the final composition for storage or for use, for example, at 1- 30wt%, or possibly for example, at 30mM to 750mM. Such ionic drugs may have multiple pKa's, which will determine where the steep slopes are on the titration curve. The present invention is especially useful in cases where the drug has a pKa that lies between the natural pH of the drug in aqueous solution and the pH range which is desirable for effective transdermal drug delivery and storage stability. The ion exchange is used to adjust the pH across the pKa before buffering with a multipeptide. Typically, for a cationic drug, the pH is first adjusted with an anion exchange material before buffering with a multipeptide buffer; for an anionic drug, the pH is first adjusted with a cationic exchange material before buffering with a multipeptide buffer. Cationic drugs that can be used in the methods of the invention include any cationic drug that, when present in a formulation, has a first pKa, or any subsequent pKa, that is lower than the pH of the formulation buffering range. Certain cationic drugs may have at least one pKa that is above the pH of the formulation buffering ranges. With cationic drugs, the only case in which there can only be one pKa for the drug would be where the pKa is higher than the formulation pH. However, the cationic drug may have at least one pKa that is lower than the desired storage or electrotransport operating pH and have at least one pKa that is higher than the storage or electrotransport operating pH. Examples of such cationic drugs that have multiple pKa's include, but are not limited to, 2-[3-[4-(4-piperidinyloxy)anilino]-1propenyl]benzamidine derivatives such as, for example, ([3-(3-Carbamimidoyl-phenyl)-2-fluoro-alllyl]-{4-[1-(1-imino-ethyl)-piperidin-4-yloxy]-phenyl}-sulfamoyl)-acetic acid hydrochloride; ([3-(3-Carbamimidoyl-phenyl)-2-methyl-allyl]- {4-[1-(1-imino-ethyl)-piperidin-4-yloxy)-phenyl}-sulfamoyl)-acetic acid hydrochloride; and ([3-(3-Carbamimidoyl-phenyl)-2-fluoro-allyl]- {3-carbamoyl-4-[1-(1-imino-ethyl)-piperidin-4-yloxy]-phenyl}-sulfamoyl)-acetic acid hydrochloride.

Anionic drugs that can be used in the methods of the invention include any anionic drug that, when present in a formulation, has a first pKa, or any subsequent pKa, that is lower than the pH of the formulation buffering range. Such a drug may have only one pKa. However, the anionic drug may have at least one pKa that is higher than the desired storage or electrotransport operating pH and have at least one pKa that is lower than the storage or electrotransport operating pH. Examples of such anionic drugs include, but are not limited to, captopril and lisinopril.

As used herein, the terms "separating," "separate," and all variations thereof refer to removing substantially all of the first ion exchange material from the drug solution.

As used herein, the terms "adding," and "add," and all variations thereof, refer to any means that directly or indirectly cause placement together of moieties or components, such that the moieties or components come into close proximity to each other. The terms include acts such as placing the moieties or components together in a container, combining the moieties or components, contacting the moieties or components, or stirring, vortexing, or agitating the moieties or components together.

As used herein, the terms "ion exchange resin" or "ion exchange material" refers to any material comprising (i) a mobile ionic species selected from the group consisting of hydronium and hydroxyl ions, and (ii) at least one oppositely charged, substantially immobile ionic species. The ion exchange materials useful in conjunction with certain embodiments of the invention are capable of donating either a hydroxyl ion (i.e., anion exchange materials or resins, which are typically used to adjust the pH of cationic drug formulations), or a hydrogen ion (i.e., cation exchange materials or resins, which are typically used to adjust the pH of anionic drug formulations).

As used herein, the terms "electrotransport" and "electrically-assisted transport" are used to refer to the delivery of drugs by means of an applied electromotive force to a drug-containing reservoir. The drug can be delivered by electromigration, electroporation, electroosmosis or any combination thereof. Electroosmosis has also been referred to as electrohydrokinesis, electroconvection, and electrically induced osmosis. In general, electroosmosis of a species into a tissue results from the migration of solvent in which the species is contained, as a result of the application of electromotive force to the therapeutic species reservoir, i.e., solvent flow induced by electromigration of other ionic species. During the electrotransport process, certain modifications or alterations of the skin can occur such as the formation of transiently existing pores in the skin, also referred to as "electroporation". Any electrically assisted transport of species enhanced by modifications or alterations to the body surface (e.g., formation of pores in the skin) are also included in the term "electrotransport" as used herein. Thus, as used herein, the terms "electrotransport" and "electrically-assisted transport" refer to (1) the delivery of charged drugs by electromigration, (2) the delivery of uncharged drugs by the process of electroosmosis, (3) the delivery of charged or uncharged drugs by electroporation, (4) the delivery of charged drugs by the combined processes of electromigration and electroosmosis, and/or (5) the delivery of a mixture of charged and uncharged drugs by the combined processes of electromigration and electroosmosis. The term "electrotransport delivery system" refers to any device that can be used to perform electrotransport.

As used herein, the term "competing ions" refers to ionic species having the same sign charge as the drug to be delivered by electrotransport, and which may take the place of the drug and be delivered through the body surface. Similarly, conventional buffering agents used to buffer the pH of a donor reservoir solution can likewise result in the addition of competing ions into the donor reservoir, which results in lower efficiency of electrotransport drug delivery.

The term "gel matrix," as used herein, refers to a composition that the reservoir of an electrotransport delivery device generally contains.

As used herein, the terms "reducing," "reduce," and all variations thereof, when used in connection to pH, refer to decreasing by any measurable degree variations in the pH of a drug solution.

As used herein, the term "delivering" refers to the administration of a drug to a patient or test subject using electrotransport.

The term "patient," as used herein, refers to an animal, mammal, or human being. The term "multipeptide" denotes any polypeptidic chain of 2 to 5 amino acid residues. The term encompasses dipeptides, tripeptides, tetrapeptides, and pentapeptides, and particularly includes multipeptides, such as dipeptides and tripeptides, that contain His, such as but not limited to, His-Gly, Gly-His, Ala-His, His-Ser and His-Ala.The term "multipeptide buffer" or "peptidic buffer" refers to buffer that contains any polypeptidic chain of 2 to 5 amino acid residues that has buffering capacity. The term encompasses dipeptides, tripeptides, tetrapeptides, and pentapeptides, and particularly includes multipeptides, such as dipeptides and tripeptides, that contain His, such as but not limited to, His-Gly, Gly-His, Ala-His, His-Ser and His-Ala.

The present invention relates to methods for preparing compositions for use in electrotransport delivery systems. The methods reduce changes in the pH of drug solutions during electrically assisted transport and during long-term storage without introducing competing ions that could negatively impact flux, resulting in effective delivery and stability of the drugs. The methods also prevent catalysis of drugs that have poor stability outside certain pH ranges by maintaining the pH of solutions of such drugs at a desired level. In certain embodiments, the methods of the invention involve a two-step process in which the pH of a drug solution is first adjusted using ion exchange means that avoid or minimize the introduction of competing ions into the solution, and then a buffering agent is added to the pH-adjusted drug solution, which reduces changes in the pH of the drug solution during electrotransport or storage. In certain embodiments of the invention, the buffering agent is a peptidic buffer. In certain embodiments of the invention, the buffering agent used in the second step of the process is an ion exchange polymeric resin. In yet certain embodiments, the buffering agent used in the second step of the process is a combination of peptidic buffer and polymeric ion exchange resin.

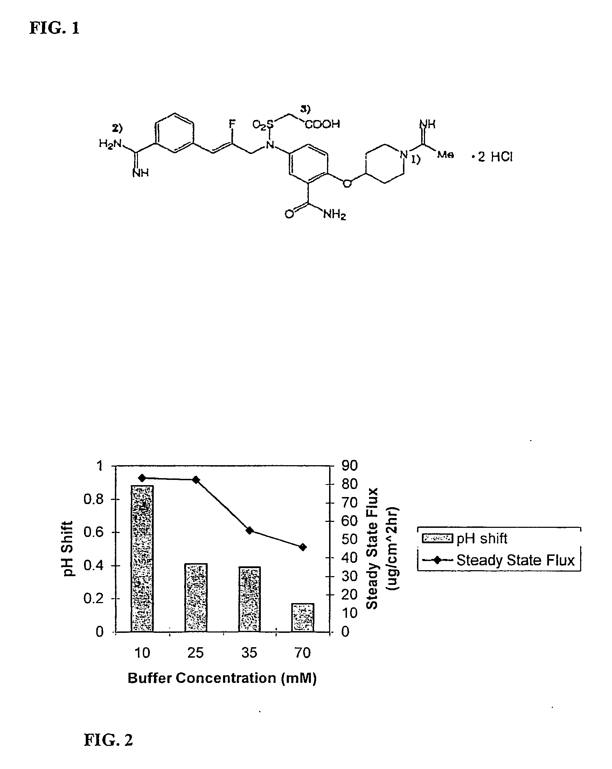

In certain embodiments of the invention, the drug is a cationic or anionic factor Xa inhibitor and an anti-coagulant. In preferred embodiments, the drug is a cationic benzamidine or naphthamidine derivative. In more preferred embodiments, the drug is a cationic benzamidine derivative. In still more preferred embodiments, the drug is a 2-[3-[4-(4-piperidinyloxy)anilino]-1propenyl]benzamidine derivative as described, for example, in Japanese Patent Number JP 2003002832 and PCT Application Publication Number WO 02/089803, incorporated herein by reference in their entireties. In even more preferred embodiments, the drug is the 2-[3-[4-(4-piperidinyloxy)anilino]-1propenyl]benzamidine derivative depicted in FIG. 1 and referred to as ROH-4746. In certain embodiments of the invention, the drug is an anionic drug, such as, for example, captopril or lisinopril. In other embodiments of the invention, the drug is terbutaline.

Divalent and polyvalent drugs that can be used in certain embodiments of the methods and compositions of the invention include, but are not limited to, alniditan, as well as talipexole dihydrochloride, carpipramine dihydrochloride, histamine dihydrochloride, proflavine dihydrochloride and gusperimus trihydrochloride. The concentration of the drug in the formulations prepared by the methods of certain embodiments of the invention depends upon the delivery requirements for the drug. The percent drug loading can range, for example, from about 1 % to about 30 %, more preferably from about 1.5 % to about 20 %, and more preferably from about 2 % to about 10 % by weight.

As mentioned, in certain embodiments, the present invention is directed to methods that involve an initial adjustment of the pH of a drug solution prior to incorporating the solution into an electrotransport drug delivery system. The pH of any particular drug solution can be adjusted either upward or downward, as desired. In this way, the flux of the drug through the skin can be optimized, as can the stability of particular drug/polymer matrix compositions. In this regard, it has been found that partially or completely neutralized drug solutions can yield a higher transdermal flux than the corresponding drug salt formulation, particularly when the drug is a divalent or polyvalent species.

In contrast to prior methods used to adjust the pH of donor drug solutions prior to electrotransport delivery, the present technique avoids, or minimizes, the introduction of extraneous ions into the electrotransport system that would compete with the drug ions for electrotransport through the body surface. For example, with cationic drugs, partial or complete neutralization by admixture with potassium hydroxide, sodium hydroxide, or the like would result in the incorporation of potassium ions, sodium ions, or the like, into the drug formulation, species that would in turn compete with the cationic drug for electrotransport delivery and reduce the efficiency of drug delivery. By adjusting the pH without, or with a reduced amount of such alkali, such competing ions can be avoided or the amount thereof minimized.

In certain embodiments, the present invention relates to methods in which the pH of a drug solution comprising drug ions and associated counterions is adjusted prior to incorporating the solution into an electrotransport drug delivery system. In certain embodiments of the invention, the pH of the drug solution is first adjusted by contacting the drug solution with an ion exchange material (first ion exchange material). In certain aspects, the invention is directed to methods in which the drug ions are cationic, the associated counterions are anionic, and the first ion exchange material is a polymeric anion exchange material. In preferred embodiments of the invention, the first polymeric anion exchange material is a polymeric anion exchange resin or a polymeric anion exchange membrane. Likewise, anionic drugs have cationic counterions and the first polymeric material for first adjusting the drug solution is preferably a polymeric cation exchange resin or a polymeric cation exchange membrane.

With cationic drugs, in certain embodiments of the invention, the first ion exchange material (i.e., the ion exchange material first used to adjust the pH of the drug solution) is preferably a polymeric anion exchange material that will exchange hydroxyl ions for the negatively charged counterions typically associated with cationic drugs, e.g., chloride, bromide, acetate, trifluoroacetate, bitartrate, propionate, citrate, oxalate, succinate, sulfate, nitrate, phosphate, and the like. Suitable anion exchange materials are typically the hydroxide forms of amine-containing polymers, e.g., polyvinyl amines, poly epichlorohydrin/tetraethylenetriamines, polymers containing pendant amine groups, and the like. A preferred anion exchange material for use herein is a co-polymer of styrene and divinyl benzene having quaternary ammonium functionality and an associated hydroxyl ion. Other suitable anion exchange materials include, but are not limited to, the hydroxide forms of Amberlite^{™} IRA-958 (an acrylic/divinylbenzene copolymer available from Rohm and Haas), cholestyramine (a styrene/divinylbenzene copolymer also available from Rohm and Haas), Dowex 2X8 (a styrene/divinylbenzene available from Dow Chemical), and Macro-Prep High Q (an acrylic/ethyleneglycol dimethacrylate copolymer available from BioRad Laboratories). As will be appreciated by those skilled in the art, anion exchange materials containing primary, secondary and tertiary amines are relatively weak bases, while those containing quaternary amine functionalities are strongly basic, and will more quickly and effectively adjust upward the pH of formulations of cationic drug salts. Accordingly, such materials are preferred for use herein.

In certain aspects, the invention is directed to preparing compositions in which the drug ions are anionic, the associated counterions are cationic, and a polymeric cation exchange material is first (first ion exchange material) used to adjust the pH of the drug prior to buffering. In preferred embodiments of the invention, the first polymeric cation exchange material is a polymeric cation exchange resin or a polymeric cation exchange membrane.

With anionic drugs, in certain embodiments of the invention, the first ion exchange material used is preferably a cation exchange material that will exchange hydrogen or hydronium ions for the positively charged counterions typically associated with anionic drugs. Salts of cationic drugs are usually formed by treating the free acid form of the drug with a pharmaceutically acceptable base, typically an amine such as diethylamine, triethylamine, ethanolamine, or the like, giving rise to positively charged quaternary ammonium moieties associated with the drug. Cation exchange resins that will exchange hydrogen ions for such species include, for example, cation exchange resins comprising a polymer having one or more acid moieties. Such polymers include, for example, polyacrylic acids, polyacrylic sulfonic acids, polyacrylic phosphoric acids and polyacrylic glycolic acids. Cation exchange resins containing carboxylic acid moieties are weaker acids and are relatively more useful for buffering, while those containing functionalities such as sulfonic acids are more strongly acidic, and are accordingly preferred in connection with the present methods as providing faster and more efficient pH adjustment. Cation exchange resins of weaker acids useful for buffering include Amberlite IRP-64 (from Rohm and Haas) and acrylic polymers such as Bio-Rex 70 from Biorad.

When preparing drug solutions adapted for electrotransport delivery through human skin, the preferred direction and type of pH adjustment will depend upon whether the drug is cationic, and hence delivered from an anodic reservoir, or anionic and hence delivered from a cathodic reservoir, as well as on the solubility characteristics of the particular drug to be delivered. In general for electrotransport delivery through human skin, the pH of an anodic reservoir formulation is typically in the range of about 4 to about 10, more preferably in the range of about 5 to about 8, and most preferably from about 6 to about 7. In general for electrotransport delivery through human skin, the pH of a cathodic reservoir is typically in the range of about 2 to about 6, more preferably in the range of about 3 to about 5.

It will be appreciated by those skilled in the art that conventional ion exchange materials used as the first ion exchange material in the methods of the invention, e.g., cation and anion exchange resins, may be replaced with any relatively high molecular weight material having acid or base functionalities, such that conversion of ionized functionalities present in the drug molecule will be effected by exchange with protons or hydroxyl ions present in the material, and separation of the drug solution therefrom will be facilitated by virtue of the material's molecular weight. Generally, although not necessarily, it is preferred that the molecular weight of the material be at least about 200 Daltons, more preferably at least about 300 Daltons, and most preferably at least about 500 Daltons.

In certain embodiments, the present invention relates to methods in which the pH of a drug solution comprising drug ions and associated counterions is adjusted prior to incorporating the solution into an electrotransport drug delivery system. In certain embodiments of the invention, the pH of the drug solution is adjusted by contacting the drug solution with a first ion exchange material. In certain embodiments, the drug solution is contacted with the first ion exchange material by simple admixture of the first ion exchange material, typically in the form of an ion exchange resin associated with a solid support (e.g., beads or the like), with a solution of the drug salt. The relative quantities of the first ion exchange material and the drug salt will depend upon the desired change in pH, which is in turn dependent upon the degree of drug salt neutralization. Generally, the pH of the drug formulation will be adjusted such that the flux of the drug through the skin, during electrotransport drug delivery, is optimized. Accordingly, the preferred pH for any given drug salt formulation may be readily determined by conducting routine experimentation to evaluate optimum drug flux. For divalent or polyvalent drugs, neutralization is generally conducted to a degree effective to convert a substantial fraction of the drug salt, typically greater than about 80%, to a monovalent form.

Reaction between the drug solution and the first ion exchange material is typically quite fast, on the order of minutes. After the reaction is allowed to proceed to completion, the drug solution may be separated from the first ion exchange material using centrifugation, standard filtration techniques (e.g., filters, screens, etc.), or using a syringe and a narrow gauge (e.g., 26 gauge) needle. It is possible that there may be trace amount of ion exchange material that passes through the ion-exchanger-removal process.

The pH-adjusted drug solution can then be processed to be introduced into the reservoir of an electrotransport delivery system, typically by incorporation into a gel matrix material that serves as the drug reservoir. A buffering material can be used to maintain the pH of the pH-adjusted drug solution in storage or in application to a patient. The buffering material is preferably a multipeptide. It is preferred that the resulting drug solution contains substantially no ion exchange material such that pH buffering by the multipeptide can be better controlled. It is preferred that any ion exchange material if present in the buffered drug solution, such as that which may have passed through a filtration process, be at a concentration of 0.2wt% or less, preferably 0.1 wt% or less, more preferably 0.05wt% or less.The present invention provides a buffered aqueous formulation for transdermal electrotransport delivery exhibiting excellent stability characteristics, either in storage or in application on a patient. The reservoir formulation may be advantageously a donor reservoir formulation containing a drug or other therapeutic agent to be transdermally delivered. Of course, peptidic buffer may also be used in a formulation for a counter reservoir formulation containing an electrolyte (e.g., saline). The formulation comprises an aqueous solution of the drug or electrolyte buffered with a peptidic buffer, including one or more multipeptides, preferably dipeptide or tripeptide, especially a dipeptide. The peptidic buffer includes a polypeptidic chain of two to five amino acids, and has an isoelectric pH at which the multipeptide carries no net charge. The aqueous solution has a pH that is within about 1.0 pH unit of the isoelectric pH (i.e., pI). Preferably, the multipeptide has at least two pKa's that are separated by no more than about 3.5 pH units. Preferably, the isoelectric pH of the multipeptide is between about 3 and 10. The concentration of the peptidic buffer in the solution is preferably at least about 10mM. The multipeptide is preferably selected from the group consisting of Asp-Asp, Gly-Asp, Asp-His, Glu-His, His-Glu, His-Asp, Glu-Arg, Glu-Lys, Arg-Glu, Lys-Glu, Arg-Asp, Lys-Asp, His-Gly, His-Ala, His-Asn, His-Citruline, His-Gln, His-Hydroxyproline, His-Isoleucine, His-Leu, His-Met, His-Phe, His-Pro, His-Ser, His-Thr, His-Trp, His-Tyr, His-Val, Asn-His, Thr-His, Try-His, Gin-His, Phe-His, Ser-His, Citruline-His, Trp-His, Met-His, Val-His, His-His, Isoleucine-His, Hydroxyproline-His, Leu-His, Ala-His, Gly-His, Beta-Alanylhistidine, Pro-His, Carnosine, Anserine, Tyr-Arg, Hydroxylysine-His, His-Hydroxytlysine, Omithine-His, His-Lys, His-Ornithine and Lys-His. A particularly preferred dipeptide in the buffer is Gly-His.

The present invention also provides a method of buffering an aqueous solution of a drug or an electrolyte used for transdermal electrotransport delivery. The method includes providing in the solution a pH buffering amount of a multipeptide including a polypeptidic chain of two to five amino acids, and having an isoelectric pH at which the multipeptide carries no net charge. The aqueous solution has a pH which is within about 1.0 pH unit of the isoelectric pH. Preferably, the multipeptide has the properties as described above for the buffered aqueous formulation.

The practice of the present invention will employ, unless otherwise indicated, conventional methods of protein chemistry, electrochemistry and biochemistry within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., 1975); J.S. Newman, Electrochemical Systems (Prentice Hall, 1973); and A.J. Bard and L.R. Faulkner, Electrochemical Methods, Fundamentals and Applications (John Wiley & Sons, 1980).

It must be noted that, as used in this specification and the appended claims, the singular forms "a", "an" and "the" include plural referents unless the content clearly dictates otherwise. Thus, for example, reference to "a polypeptide" includes a mixture of two or more polypeptides, and the like.

The following amino acid abbreviations are used throughout the text:

| | |
|---|---|
| Alanine: Ala (A) | Arginine: Arg (R) |
| Asparagine: Asn (N) | Aspartic acid: Asp (D) |
| Cysteine: Cys (C) | Glutamine: Gln (Q) |
| Glutamic acid: Glu (E) | Glycine: Gly (G) |
| Histidine: His (H) | Isoleucine: Ile (I) |
| Leucine: Leu (L) | Lysine: Lys (K) |
| Methionine: Met (M) | Phenylalanine: Phe (F) |
| Proline: Pro (P) | Serine: Ser (S) |
| Threonine: Thr (T) | Tryptophan: Trp (W) |
| Tyrosine: Tyr (Y) | Valine: Val (V) |

In performing electrotransport experiments in animals, it has surprisingly been discovered that some buffers are better suited for pH control. In particular, buffer multipeptides such as Gly-His and His-Glu at their pI are capable of assuring pH control of electrotransport formulations for several hours. Multipeptide buffers for use in the present invention include dipeptides, tripeptides, tetrapeptides, and pentapeptides which contain His, such as His-Gly, Gly-His, Ala-His, L-carnosine (also known as L-Ala-His), His-Ser, His-Ala, Gly-Gly-His (pl = 7.5), His-Gly-Gly (pl = 6.9).

Preferably, the multipeptide should have at least two pKa's separated by no more than about 3.5 pH units. Beyond this range, pH control will be poor and conductivity of the solution will be minimal. The pI range of the multipeptide should be between 3 and 10 and the target pH of the formulation preferably is no more than about 1 pH unit away from the isoelectric pH (i.e., the pI) of the multipeptide. Generally, the formulation pH will be from about pH 3 to about pH 9.5. However, the preferred formulation pH will depend on the particular drug and peptidic buffer used in the formulation. Beyond these pH limits (i.e., less than pH 3 and greater than pH 10), the formulation is likely to be irritating or will result in unacceptable skin resistance. In addition, if the formulation pH is more than 1 pH unit away from the pI of the peptidic buffer, the effects described above will become less efficient as the multipeptide will start behaving like a conventional buffer (high transport efficiency of charged species and pH drifting). When the multipeptide is used in a solution having a pH at or close to the pI of the multipeptide (i.e., pI ± 1.0 pH unit), minimum competition with the drug ions (i.e., for electrotransport into the patient) will occur because the buffer is at or close to electrical (i.e., ionic) neutrality and therefore it can be used with good results (i.e., little or no ionic competition with the drug ions) in either the anode or the cathode reservoir formulations. If for technical reasons it is decided to use the multipeptide at a pH between 0.5 to 1.0 pH unit away from the pI, the use of the buffer at a pH slightly higher than its pI is preferred in the cathodic formulation in order to minimize ionic competition with the drug being delivered. Conversely, and for the same reason, the use of the peptidic buffer at a pH slightly below (i.e., between 0.5 to 1.0 pH unit below) its pI is preferred in the anodic formulation. In the counter reservoir formulation (i.e., the non-drug containing reservoir) this preference is not as important as there is no concern over the buffer ions competing with drug ions for delivery into the patient from the counter reservoir. The multipeptide will generally be present in the formulation at a concentration of from about 10 mM to 1 M, more preferably from about 10mM to about 250mM, and most preferably from about 25mM to about 100mM.

Table 1 lists conductivities and solubilities of selected multipeptides useful in the present invention, at their pI.

TABLE 1

| Multipeptide | pI | Conductivity at 10⁻² Molar (µS*/cm) | Solubility (Moles/l) |
|---|---|---|---|
| His-Glu | 5.20 | 40 | 0.40 |
| His-Asp | 5.22 | 28 | 0.05 |
| Glu-Lys | 6.00 | 6 | 1.00 |
| Lys-Glu | 6.06 | 8 | 0.50 |
| Lys-Asp | 6.08 | 6 | 1.00 |
| His-Gly | 6.90 | 40 | 1.00 |
| His-Ala | 6.95 | 60 | 0.50 |
| Val-His | 7.38 | 94 | 0.20 |
| Gly-His | 7.55 | 52 | 1.00 |

| | | | |
|---|---|---|---|
| *µS = micro Siemens | | | |

The peptidic buffer preferably includes at least one amino acid selected from His, Asp, Glu, Lys, Tyr, Arg and Cys; more preferably includes at least one amino acid selected from His, Asp, Glu, and Lys; and most preferably includes at least one amino acid selected from His and derivatives thereof (e.g., methyl-His).

Many multipeptides present adequate characteristics for use in electrotransport formulation. Table 2 includes a non-exhaustive list of the peptidic buffers ranked by increasing pI. Multipeptides having up to five amino acids and containing the amino acids histidine, lysine, aspartic acid or glutamic acid in combination or with other amino acids are particularly useful to this invention. Dipeptides and tripeptides are especially preferred.

TABLE 2

| Multipeptide | pka A | pka A | pka A | pka B | pka B | pka B | pI | % salt |
|---|---|---|---|---|---|---|---|---|
| Asp-Asp | 2.70 | 3.40 | 4.70 | 8.26 | | | 3.05 | 43 |
| Gly-Asp | 2.81 | 4.45 | | 8.60 | | | 3.60 | 23 |
| Asp-His | 2.45 | 3.02 | | 6.81 | 7.98 | | 4.90 | 3 |
| Glu-His | 2.45 | 3.45 | | 6.81 | 8.20 | | 5.20 | 5 |
| His-Glu | 2.30 | 4.19 | | 6.32 | 8.07 | | 5.20 | 15 |
| His-Asp | 2.28 | 3.99 | | 6.45 | 8.19 | | 5.22 | 11 |
| Glu-Arg | 2.66 | 4.01 | | 7.94 | 12.50 | | 6.00 | 2 |
| Glu-Lys | 2.85 | 4.01 | | 7.94 | 11.07 | | 6.00 | 2 |
| Arg-Glu | 2.74 | 4.18 | | 7.92 | 12.50 | | 6.06 | 3 |
| Lys-Glu | 2.74 | 4.18 | | 7.92 | 11.12 | | 6.06 | 3 |
| Arg-Asp | 2.64 | 4.10 | | 8.05 | 12.50 | | 6.08 | 2 |
| Lys-Asp | 2.64 | 4.10 | | 8.05 | 11.20 | | 6.08 | 2 |
| His-Gly | 2.41 | | | 5.90 | 7.91 | | 6.90 | 16 |
| His-Ala | 2.48 | | | 6.10 | 7.80 | | 6.95 | 22 |
| His-Asn | 2.62 | | | 6.10 | 7.80 | | 6.95 | 22 |
| His-Citrulline | 3.05 | | | 6.10 | 7.80 | | 6.95 | 22 |
| His-Gln | 2.93 | | | 6.10 | 7.80 | | 6.95 | 22 |
| His-Hydroxyproline | 2.42 | | | 6.10 | 7.80 | | 6.95 | 22 |
| His-Isoleucine | 3.13 | | | 6.10 | 7.80 | | 6.95 | 22 |
| His-Leu | 3.10 | | | 6.10 | 7.80 | | 6.95 | 22 |
| His-Met | 2.89 | | | 6.10 | 7.80 | | 6.95 | 22 |
| His-Phe | 2.88 | | | 6.10 | 7.80 | | 6.95 | 22 |
| His-Pro | 2.62 | | | 6.10 | 7.80 | | 6.95 | 22 |
| His-Ser | 2.65 | | | 6.10 | 7.80 | | 6.95 | 22 |
| His-Thr | 2.98 | | | 6.10 | 7.80 | | 6.95 | 22 |
| His-Trp | 3.07 | | | 6.10 | 7.80 | | 6.95 | 22 |
| His-Tyr | 2.13 | 9.97 | | 6.10 | 7.80 | | 6.95 | 22 |
| His-Val | 3.18 | | | 6.10 | 7.80 | | 6.95 | 22 |
| Asn-His | 2.42 | | | 6.71 | 7.30 | | 7.00 | 44 |
| Thr-His | 2.42 | | | 6.71 | 7.60 | | 7.15 | 39 |
| Tyr-His | 2.42 | 9.90 | | 6.71 | 7.60 | | 7.15 | 39 |
| GIn-His | 2.42 | | | 6.71 | 7.70 | | 7.20 | 36 |
| Phe-His | 2.42 | | | 6.71 | 7.70 | | 7.20 | 36 |
| Ser-His | 2.42 | | | 6.71 | 7.70 | | 7.20 | 36 |
| Citrulline-His | 2.42 | | | 6.71 | 7.90 | | 7.30 | 32 |
| Trp-His | 2.42 | | | 6.71 | 7.90 | | 7.30 | 32 |
| Met-His | 2.42 | | | 6.71 | 7.97 | | 7.35 | 30 |
| Val-His | 3.09 | | | 6.83 | 7.94 | | 7.38 | 34 |
| His-His | 2.25 | | | 5.40 | 6.80 | 7.95 | 7.40 | 32 |
| Isoleucine-His | 2.42 | | | 6.71 | 8.20 | | 7.44 | 25 |
| Hydroxyproline-His | 2.42 | | | 6.71 | 8.23 | | 7.45 | 25 |
| Leu-His | 2.42 | | | 6.71 | 8.25 | | 7.50 | 24 |
| Ala-His | 2.42 | | | 6.71 | 8.37 | | 7.55 | 22 |
| Gly-His | 2.42 | | | 6.71 | 8.39 | | 7.55 | 22 |
| Beta-Alanylhistidine | 2.60 | | | 6.70 | 8.70 | | 7.70 | 16 |
| Pro-His | 2.42 | | | 6.71 | 9.10 | | 7.90 | 11 |
| Carnosine | 2.64 | | | 6.83 | 9.51 | | 8.17 | 8 |
| Anserine | 2.64 | | | 7.04 | 9.49 | | 8.27 | 10 |
| Tyr-Arg | 2.64 | 9.36 | | 7.39 | 11:62 | | 8.40 | 17 |
| Hydroxylysine-His | 2.42 | | | 6.71 | 7.40 | 9.70 | 8.60 | 13 |
| His-Hydroxylysine | 3.05 | | | 6.10 | 7.80 | 9.70 | 8.75 | 17 |
| Ornithine-His | 2.42 | | | 6.71 | 7.30 | 11.00 | 9.20 | 3 |
| His-Lys | 3.05 | | | 6.10 | 7.80 | 11.00 | 9.40 | 5 |
| His-Ornithine | 2.82 | | | 6.10 | 7.80 | 11.00 | 9.40 | 5 |
| Lys-His | 2.42 | | | 6.71 | 8.00 | 11.00 | 9.50 | 5 |

| | | | | | | | | |
|---|---|---|---|---|---|---|---|---|
| pKa A = acidic pKa pKa B = basic pKa % salt = fraction of the multipeptide that is ionized and carries a net positive and/or negative charge, but not including the ionized species carrying a net neutral charge, in an aqueous solution having a pH equal to the pI | | | | | | | | |

The pH buffering capacity of the peptidic buffers of the present invention can be explained by using Gly-His at pH 7.5 as an example (the pI of Gly-His is 7.55). At this pH, the net charge of the molecule is essentially zero. At the pI, three species coexist. The bulk of the molecule (70%) consists of the neutral species that bears two internal charges, one positive and one negative resulting in a net charge of zero. The remaining (30%) consists of the salt form of the positively charged species (1-2+, net charge = +1) and the negatively charged species (-1). The existence of this salt can be demonstrated by measuring the conductivity of the solution of a Gly-His solution at its pI (Table 1). Although there are small percentages of species presenting a net positive or negative charge in solution, there is minimal ionic transport of these charged species due to charge equilibrium between the three species (i.e., a positive charge migrating in the electric field will revert almost instantly to its neutral form and lose momentum or to its negative form and migrate backward). Thus, there is little if any ionic transport of the charged peptides into the systemic circulation of the patient. Due to the same principle of charge equilibrium, any depletion of the charged molecules will be compensated immediately by dissociation of the neutral form to its charged species thereby providing a reservoir insuring long term pH stability. In addition, if loss of the molecule occurs by electroosmosis of the neutral species, this will not result in any pH changes.

The pH buffering capacity of multipeptides at or near their isoelectric pH exhibit better ability to maintain pH stability with less competing ions than observed with conventional buffers such as phosphate or 3-[N-morpholino] propane sulfonic acid (MOPS) at the same or higher ionic strength.

This invention is particularly useful in maintaining pH of cationic drugs such as benzamidine derivatives, e.g., ROH-4746. For buffering of benzamidine derivative, e.g., ROH-4746, a particularly useful group of zwitterionic multipeptide buffers includes, but not limited to, Asp-His, Glu-His, His-Glu, His-Asp, Glu-Arg, Glu-Lys, Arg-Glu, Lys-Glu, Arg-Asp, Lys-Glu, Arg-Asp, Lys-Asp, and His-Gly. When used at the pI to maintain pH, introduction of competitive ions into the formulation can be minimized if interactions with the drug are eliminated.

ROH-4746 is supplied as the dihydrochloride and possesses one acidic and two basic pKa's (2.6, 9.4, 11.6). At different pH's, the charge on the drug may be +2, +1, 0, or -1. In addition, aqueous stability studies indicated the long-term stability of the drug was found to be favorable in the range of 4.0-6.5 pH units. It is important to control the pH of the formulation during use to preserve the stability of ROH-4746 as well as maintain the efficiency of the system by delivering the +1 charged molecule. These two facts work well together in that the stable working pH range overlaps the pH range at which the drug molecule maintains a +1 charge. To ensure delivery of the +1 drug molecule, initial pH adjustment of the added drug solution can be done by ion exchange. This alone does not solve the problem of buffering in order to maintain the adjusted pH in storage or application to a patient. Thus, a second step of buffering is required and is accomplished with the addition of the peptidic buffer. It was also important to limit the addition of competing ions, which hinder flux, into the formulation. Furthermore, by adding the multipeptide to the drug solution, previously adjusted using the ion exchange resin, the final pH can be set at the isoelectric point without the use of traditional bases (i.e. sodium hydroxide) typically needed to shift the pH to the desired point. By eliminating the use of bases in the formulation the addition of competing ions into the formulation has effectively been eliminated.

As is well known that when a titrating agent (e.g., a base such as NaOH for titrating cationic drug) is added to a drug (e.g., a cationic drug such as ROH-4746), the pH initially changes slowly per amount of titrating agent used about a first pKa of the drug due to the buffering capacity of the drug itself at the pKa. After the pH is titrated past the first pKa, perhaps about 1 to 2 pH units, the pH shifts to a phase in which the pH changes very quickly per amount of the titrating agent used. With further addition of the titrating agent, the pH change per amount of titration agent used slows again. Thus, after titrating past the first pKa, there is a steep slope with an inflection point in, the titration curve. This steep slope is therefore proximate to the first pKa and in the direction of titration. For ROH-4746, there is such a steep slope on the neutral side of the first (lowest) pKa. For drugs with multiple pKa's, such as ROH-4746, there may be multiple inflection points along the titration curve. A first steep slope of fast pH change about an inflection point for ROH-4746 is about pH 4.0 to 8.0, past the first pKa of 2.6, but below the second pKa of 9.4. There is another inflection point above the second pKa of 9.4. Likewise, for an anionic drug, the titration of the anionic drug with acid (e.g., HCl) from a high pH to a low pH will have a steep slope about an inflection point on the titration curve after titrating past a first pKa, if the highest pKa is considered to be the first pKa for an anionic drug. For anionic drug with multiple pKa's, there will likewise be multiple inflection points. Such titration curves for cationic or anionic drugs can be obtained by titrating with typical acids and bases (such as NaOH, HCl and the like), and can also be buffered with multipeptides of appropriate pH and pl. Titration can be done in any pH direction to achieve the charge and stability region of interest.

An inflection point on a titration curve can be determined by traditional methods known in the art of titration. For the purpose of this invention to determine the range with a steep slope, an inflection point on the titration curve can be obtained by measuring the slope of points along the curve and selecting the point with the steepest slope. This can be done mathematically, using a computer algorithm, or graphically by hand. Alternatively, for a drug with more than one pKa's, to obtain the inflection point between two pKa's, one can consider the pH midpoint between the two pKa's as the inflection point therebetween. Yet another way to determine an inflection point is to determine the slope at various points along the curve and select a point with the fastest increase of slope and its neighboring point with the fastest decrease of slope and taking the pH midpoint between them. The range of pH between the point of the fastest increase in slope to the point of the fastest decrease in slope about that inflection point can be taken as the area of the steep slope. For practical reasons in measuring the slopes, depending on the selection of the drug, a range of about 4 pH units wide, preferably about 3 pH units wide, more preferably about 2 pH units wide, and even more preferably about 1 pH unit wide centered about the inflection point can be considered the steep slope.

The pI of the multipeptide is selected such that it is within an acceptable pH range for stability of the drug, either for storage or for application to an individual over time, or both. This range can be within a range of about 2 to 3 pH units wide for certain drugs (e.g., from a pH of about 4 to 6.5 for ROH-4746). The target pH range of the composition is selected to be in the acceptable stable pH range. Further, to select the multipeptide for buffering a specific drug, one could note the first pKa of the drug and the steep slope range past the first pKa on the titration curve and select a multipeptide with a pI that falls on the steep slope. Preferably, one would select the pI such that it is near the inflection point of that slope. Generally, the pI of the multipeptide can be selected to fall in a pH range of about 1.5 pH units on each side, preferably about 1 pH unit on each side, and more preferably about 0.5 pH unit on each side, of the inflection point on the slope. For example, it is found that ROH-4746 is stable between about pH 4 and 6.5. It is preferred that a target pH for the buffered drug composition be in the range of the stable pH range and within about 1 pH unit on either side of the pI of the multipeptide used for buffering. Thus, if a dipeptide buffer of pI=5.2 is selected, which is within 1pH unit of the inflection point, it is preferred that the target pH of the buffered drug composition be in the range of 4.2 to 6.2, which is within the stable range of ROH-4746.

To reduce the amount of competing ions resulting from the peptidic buffer, the pH of the drug solution is adjusted by ion exchange to be near or at the pI of the multipeptide, preferably within about 1.5 pH units on each side, preferably within about 1 pH unit on each side, more preferably within about 0.5 pH unit on each side, and even more preferably within about 0.25 pH unit on each side, of the pI. Further, one would select the multipeptide concentration in the composition to be used to result in desirable ranges of steady state flux with acceptable pH stability in application. Generally, pH shift of less than about 1 pH unit is preferred during use of the drug delivery device. Generally, the multipeptide concentration is selected such that the steady state flux is within about 50% of the maximum steady state flux. For example, for ROH-4746, one would select a multipeptide concentration to result in steady state flux of the drug from about 20 to 120 µg/cm²hr, preferably about 40 to 100 µg/cm²hr. Electrotransport flux tests were performed using custom built modified Franz diffusion cells that had a silver foil anode and a silver chloride cathode. The equipment set up was based on modifying a typical Franz diffusion cell well known in the art to accommodate formulations with poly(vinyl alcohol) polymer (PVOH) and to provide a constant source of fresh receptor solution. The overall housing is constructed of Delran Teflon. The anodic compartment contained the drug-containing PVOH hydrogel. The cathode compartment has a human heat separated skin contacting the PVOH hydrogel. The cathode compartment contained a receptor solution for receiving the drug that passes through the skin. The electrodes were connected to a DC power source that supplied a constant electric current of 0.100 mA/cm². Hydrogels were made with a method described in the Examples below,

As a result of using a first ion exchanger to adjust the drug solution, removing the first ion exchanger from the drug solution and buffering the resulting drug solution with peptidic buffering, a buffered drug solution with storage and electrotransport pH stability with minimal competing ions can be obtained. For example, using the methods of the present invention, in the case of ROH-4746, the resulting buffered drug solution can have a ROH-4746 concentration of about 1 to 30wt%, preferably about 2 to 10wt%, with a pH on the steep slope of the titration curve of the drug, of a range from about pH 4 to 7, preferably about 5 to 6, with a strong base cation (e.g., as alkali cation Na⁺, K⁺, or even less strong base cations such as NH₄⁺) concentration of preferably less than about 75mM, preferably less than about 40mM, and more preferably less than about 20mM. In a preferred embodiment, ROH-4746 is at a concentration of 30mM to 750mM in the buffered formulation. Other drugs can be pH-adjusted and buffered to desired pH to have similar characteristics in drug and ion concentrations.

The concentration of a buffering multipeptide or a mixture of multipeptides is generally from about 10mM to 250mM, preferably from about 20 mM to 100mM, more preferably from about 25 mM to 70mM. It is preferred that the drug containing composition is one that is purely buffered by the peptidic buffer or adds further buffering capacity to a drug buffered in a range that has some buffering capacity provided by the drug itself if the buffering range is close to a pKa of the drug. Thus, it is preferred that a drug containing composition would minimize both buffer concentration and competing ion concentration with the lowest drug concentration that doesn't hinder steady state flux.

The advantages over the prior methods of pH adjustment are not limited to the family of benzamidine derivatives, but to any situation in which the formulation buffering pH is more near a desired pH of the drug (e.g., the neutral point) than the pKa of the drug. This is applicable to all cationic drugs with at least one pKa lower than the desired storage pH and contain pKa value(s) that are higher than storage pH conditions. This invention is also applicable to anionic drugs with at least one pKa higher than the desired storage pH and contain pKa value(s) that are lower than storage pH conditions.

If desired, the ion-exchanged pH-adjusted drug solution can be buffered with just an ion exchange material without using peptidic buffers, as will be described in more detail later. Further, if desired, the ion-exchanged pH-adjusted drug solution can be buffered with both a peptidic buffer and an ion exchange material. Based on the present description on the use of peptidic buffer and ion exchange, a person skilled in the art will be able to use each for desired results.

As mentioned before, certain embodiments of the invention relate to preparing compositions for use in an electrotransport delivery system in which the pH-adjusted drug solution is contacted with a second ion exchange material. In some embodiments of the invention, the pH-adjusted drug solution is first added to the reservoir of an electrotransport delivery system, and the second ion exchange material is then added to the reservoir containing the drug solution. In other embodiments of the invention, the pH-adjusted drug solution is first contacted with the second ion exchange material, and the resultant mixture is then added to the reservoir of an electrotransport delivery system. In still other embodiments of the invention, the second ion exchange material is first added to the reservoir of an electrotransport delivery system, and then the pH-adjusted drug solution is added to the reservoir containing the second ion exchange material.

In certain embodiments of the invention, the drug ions are cationic, the associated counterions are anionic, and the second ion exchange material is a polymeric anion or cation exchange material. As understood by those of ordinary skill in the art, the choice of the appropriate second polymeric ion exchange resin is determined by the acid/base properties of the resin. For certain formulations prepared according to the methods of the invention, a polymeric anion exchange material provides the desired properties, and for certain other formulations prepared according to the methods of the invention, a polymeric cation exchange material provides the desired properties.

In certain embodiments of the invention, the drug ions are anionic, the associated counterions are cationic, and the second ion exchange material is a polymeric anion or cation exchange material. Again, as understood by those of ordinary skill in the art, the choice of the appropriate second polymeric ion exchange resin is determined by the acid/base properties of the resin.

In certain embodiments, the second polymeric anion or cation exchange material is a polymeric anion or cation exchange resin. In other embodiments, the second polymeric anion or cation exchange material is a polymeric anion or cation exchange membrane.

Suitable second polymeric cation exchange resins include, for example, polacrilin, acrylate, methyl sulfonate, methacrylate, carboxylic acid functional groups, sulfonic acid, sulfoisobutyl, or sulfoxyethyl. In particularly preferred embodiments, the second polymeric cation exchange resin includes polacrilin or acrylate.

Suitable second polymeric cation exchange resins include, for example, a polymer having one or more acid moieties. Such polymers include, for example, polyacrylic acids, polyacrylic sulfonic acids, polyacrylic phosphoric acids and polyacrylic glycolic acids. For buffering using anion exchangers, those exchange materials identified as weak anion are preferred. Several such polymers are available within the "Bio-Rex" and "AG" family of resins from Biorad (i.e. Bio-Rex 5 and AG 4-X4). Other anion exchange materials include forms of Amberlite (available from Rohm and Haas), e.g., Amberlite IRA67. Further anion exchange resins include the "Dowex" family of resins from Dow Chemicals (Dowex Monosphere 77).

The degree of neutralization of the second ion exchange resin in the formulations prepared according to certain embodiments of the methods of the invention, and the concentration of the second ion exchange resin, are spread over a range of values. In certain embodiments of the invention, the degree of neutralization of the second polymeric anion or cation exchange resin is about 2 % to about 70 %. In more preferred embodiments, the degree of neutralization of the second polymeric anion or cation exchange resin is about 5 % to about 50 %. In even more preferred embodiments, the degree of neutralization of the second polymeric anion or cation exchange resin is about 5 % to about 30 %. The percent neutralization affects the performance of the resin to act like a buffer. Percent neutralization refers to the amount of acid groups neutralized when adding (in this case) a base. For example, if 10 mmole of NaOH is added to 20 mmole of acetic acid, 50% of the acetic acid is neutralized. The degree of neutralization of the ion exchange resin was achieved by neutralizing a polymeric ion exchange resin buffer with NaOH. Data show that the percent neutralization played a role in the buffering capabilities of the polymeric buffer.

The concentration of the second polymeric anion or cation exchange resin in the formulations prepared according to certain embodiments of the methods of the invention is about 20 meq/mL to about 200 meq/mL. In more preferred embodiments, the concentration of the second polymeric anion or cation exchange resin is about 20 meq/mL to about 140 meq/mL. In even more preferred embodiments, the concentration of the second polymeric anion or cation exchange resin is about 25 meq/mL to about 60 meq/mL. The lower end of the percentage of the second ion exchange resin is preferred because it adds the least amount of competing ions (such as sodium ion) to the formulation. Furthermore, at lower concentrations, adverse effects in achieving steady state flux are avoided with shorter rise time to attain steady state flux.

In certain preferred embodiments of the invention, the drug is a 2-[3-[4-(4-piperidinyloxy)anilino]-1propenyl]benzamidine derivative and the second ion exchange resin is polacrilin. The desired amount of the polacrilin in the formulations prepared according to such embodiments of the methods of the invention is about 0.23 % to about 1.13 % by weight, neutralized to between about 5 % to about 10 %. For tighter pH control during delivery, in certain embodiments of the invention, this range would narrow down to about 0.23 % to about 0.40 % by weight of the second anion exchange resin, neutralized to between about 5 % and 8 %.

It will be appreciated by those working in the field that formulations prepared by the present methods can be used in conjunction with a wide variety of electrotransport drug delivery systems (including iontophoresis drug delivery systems), as the methods are not limited in any way in this regard. For examples of electrotransport drug delivery systems and iontophoresis drug delivery systems, reference may be had to U.S. Pat. Nos. 5,147,296 to Theeuwes et al., 5,080,646 to Theeuwes et al., 5,169,382 to Theeuwes et al., and 5,169,383 to Gyory et al, the disclosures of each of which are incorporated by reference herein in their entireties.

The reservoir of the electrotransport delivery devices generally comprises a gel matrix, with the drug solution uniformly dispersed in at least one of the reservoirs. Suitable polymers for the gel matrix can comprise essentially any nonionic synthetic and/or naturally occurring polymeric materials. A polar nature is preferred when the active agent is polar and/or capable of ionization, so as to enhance agent solubility. Optionally, the gel matrix can be water swellable. Examples of suitable synthetic polymers include, but are not limited to, poly(acrylamide), poly(2-hydroxyethyl acrylate), poly(2-hydroxypropyl acrylate), poly(N-vinyl-2-pyrrolidone), poly(n-methylol acrylamide), poly(diacetone acrylamide), poly(2-hydroxylethyl methacrylate), poly(vinyl alcohol) and poly(allyl alcohol). Hydroxyl functional condensation polymers (i.e., polyesters, polycarbonates, polyurethanes) are also examples of suitable polar synthetic polymers. Polar naturally occurring polymers (or derivatives thereof) suitable for use as the gel matrix are exemplified by cellulose ethers, methyl cellulose ethers, cellulose and hydroxylated cellulose, methyl cellulose and hydroxylated methyl cellulose, gums such as guar, locust, karaya, xanthan, gelatin, and derivatives thereof. Ionic polymers can also be used for the matrix provided that the available counterions are either drug ions or other ions that are oppositely charged relative to the active agent.

In certain embodiments of the invention, the reservoir of the electrotransport delivery system comprises a hydrogel. In other embodiments of the invention, the reservoir comprises a non-hydrogel, dry matrix.

In certain preferred embodiments of the invention, the reservoir of the electrotransport delivery system comprises a polyvinyl alcohol hydrogel as described, for example, in U.S. Patent No. 6,039,977, incorporated herein by reference in its entirety. Polyvinyl alcohol hydrogels can be prepared, for example, as described in U.S. Patent No. 6,039,977. The weight percentage of the polyvinyl alcohol used to prepare gel matrices for the reservoirs of the electrotransport delivery devices, in certain embodiments of the methods of the invention, is about 10 % to about 30 %, preferably about 15 % to about 25 %, and more preferably about 19 %. Preferably, for ease of processing and application, the gel matrix has a viscosity of from about 1,000 to about 200,000 poise, preferably from about 5,000 to about 50,000 poise.

Incorporation of the drug solution into the gel matrix can be done any number of ways, *i.e*., by imbibing the solution into the reservoir matrix, by admixing the drug solution with the matrix material prior to hydrogel formation, or the like.

Thus, after adjusting the pH of the drug solution using the methods of the invention and either before or after buffering the pH-adjusted solution, the solution is incorporated into the drug reservoir, e.g., a gel matrix as just described, and is then administered to a patient using an electrotransport drug delivery system. The buffering material (either a peptidic buffer, a second ion exchange material, or a combination thereof) serves to reduce changes in the pH of the drug reservoir, and to maintain the desired pH of the reservoir, during electrotransport, resulting in greater stability and enhanced delivery of the drug.

In certain embodiments of the invention, a pH-adjusted drug solution is buffered by mixing with a buffer (such as peptidic buffer, second ion exchange material, or combination), and the solution is then stored, rather than being incorporated into the reservoir of an electrotransport delivery device for administration to a patient. The buffer material serves to reduce changes in the pH of the drug solution and to maintain the desired pH of the solution during long-term storage. Drug solutions formulated according to the methods of the invention can thus be stably stored for extended periods of time such as, for example, weeks to months to years, depending on the selection of buffering material and conditions.

In certain other embodiments of the invention, a pH-adjusted drug solution is introduced into the reservoir of an electrotransport delivery system, and is buffered with an appropriate buffer (such as peptidic buffer, second ion exchange material, or combination), either before or after incorporation into the reservoir. Instead of being used immediately, the reservoir is stored for an extended period of time. The buffer material serves to reduce changes in the pH of the reservoir and to maintain the desired pH of the reservoir during storage. Reservoirs containing a pH-adjusted drug solution prepared according to the methods of the invention can be stably stored for extended periods of time such as, for example, weeks to months to years.

The following examples are illustrative of certain embodiments of the invention and should not be considered to limit the scope of the invention.

### EXAMPLE 1: Preparation of Cationic Drug Formulations for Electrotransport

The pH of a concentrated solution of a 2-[3-[4-(4-piperidinyloxy)anilino]-1propenyl]benzamidine derivative depicted in FIG. 1, and referred to as ROH-4746, was adjusted by adding either NaOH or small quantities of a hydroxylated anion exchange resin (AG1-X8, available from Biorad, 2000 Alfred Nobel Dr., Hercules, CA 94547) to the drug solution. The natural pH of the benzamidine derivative in water is lower than the lowest pKa of the drug. Exchange of the chloride counterion of the drug molecule with hydroxide from the resin raised the pH of the drug solution without introducing any competing ion that could reduce drug flux during electrotransport. After the pH of the drug solution was adjusted to the desired value, at or near the pI of the multipeptide of interest (e.g., His-Glu with pI = 5.2), the resin was removed by filtration through a syringe filter (0.2 µm). The multipeptide buffer, e.g., His-Glu, was then added into the drug solution to result in a concentration of 10-70 mM. The results showed that adding His-Glu of to result in a concentration of 25 mM produced better results.

Hydrogels were typically prepared by placing polyvinyl alcohol (PVOH) at 19 wt % in purified water at 90°C for 30 minutes, reducing the temperature to 50°C, dispensing the gel suspension into disks, and freeze-curing. A 100mg/mL ROH-4746 solution was prepared by dissolving the drug in purified water. The ROH-4746 solution was pH adjusted with hydroxylated ion exchange resin AG 1x8 (BioRad) to the pI of about 5.2 of peptidic buffer His-Glu and the peptidic buffer was added to the pH-adjusted ROH-4746 solution. The previously formed hydrogels were partially dehydrated and then allowed to imbibe the pH-adjusted drug solution as a concentrated aqueous solution at room temperature to obtain the desired drug loading. The amount of peptidic buffer added was adjusted to achieve the desired molar concentration of the formulation. The adjustment can be done before imbibing by the hydrogels through calculating and experimental procedure. Alternatively, the adjustment can be done by fine-tuning with further buffer addition after imbibing.

### EXAMPLE 2: In vitro Electrotransport Studies

Prepared hydrogels (as described above) were allowed to imbibe the buffered ROH-4746 solution 12 - 24 hours before experimentation allowing the drug to equilibrate throughout the gel. With the use of an intial ROH-4746 concentration of 100mg/mL before ion exchanging, the resultant ROH-4746 concentration in the hydrogels after imbibing were about 30mg/mL on aqueous basis. Electrotransport flux tests were performed as described above using modified Franz diffusion cells that had a silver foil anode and a silver chloride cathode. The modified Franz diffusion cells accommodated formulations with poly(vinyl alcohol) polymer (PVOH) provided a constant source of fresh receptor solution. The surface area of testing across which drug was passed was about 1.3 cm². The hydrogel thickness used in the test was about 1.6 mm. The overall housing was constructed of Delran Teflon. The anodic compartment contained the drug-containing PVOH hydrogel. The cathode compartment had a human heat separated skin contacting the PVOH hydrogel. The flux of ROH-4746 through the human heat separated skin was measured. The initial pH of each gel were measured prior to applying a current density of 100 µA/cm² for 24 hours. Receptor solution was analyzed by HPLC to determine drug flux. After the 24 hours, the pH was measured again to assess the buffering capacity of the multipeptide. Certain hydrogel samples were buffered with peptidic buffers and certain hydrogel samples were control samples and were not buffered with a peptidic buffer.

With multipeptides incorporated into the formulation, hydrogel pH shifts were minimized when ionic strengths of 25 mM or higher were used. Hydrogels were buffered with His-Glu (pI = 5.2) with initial pH's of approximately 5.2. The mM concentration of the multipeptide was determined by the concentration of the buffer in the aqueous components for the resulting hydrogel system (this would exclude the polyvinyl alcohol material for forming the gel matrix). FIG. 2 shows how the increase in buffer concentration in the formulation affects the steady state flux during iontophoretic use. The figure shows that there is an optimal range of concentration that will sufficiently hold pH while maintaining acceptable flux. The optimal concentration can be determined by one skilled in the art based on the choice of the buffer and the drug in view of the present disclosure.

Table 3 shows His-Glu buffered hydrogels before and after 24 hours of iontophoretic use at varied buffer concentrations. At a peptidic buffer concentration of 10 mM or 25 mM, the steady state flux was quite high, about 80 µg/cm²hr. As the peptidic buffer concentration increased further, the steady state flux suffered. However, using a higher peptidic buffer concentration reduced the tendency for pH to shift in electrotransport use over time. Thus, in this case, the optimal range for the His-Glu buffer was about 10 mM to 35mM. Among the concentrations of 10mM, 25mM, 35 mM and 70mM, the best flux was obtained with 10mM but the optimal concentration in view of good flux and low pH shift was about 25mM.

**Table 3: Initial and Post Flux Anode Hydrogel pH's Containing ROH-4746 Buffered with His-Glu**

| Buffer Concentration (mM) | Initial pH | Final pH |
|---|---|---|
| 10 | 5.21 | 6.17 |
| 10 | 5.19 | 5.99 |
| 25 | 5.17 | 5.79 |
| 25 | 5.17 | 5.37 |
| 35 | 5.19 | 5.83 |
| 35 | 5.14 | 5.53 |
| 35 | 5.19 | 5.54 |
| 70 | 5.23 | 5.41 |
| 70 | 5.19 | 5.36 |

### EXAMPLE 3: Long Term Storage Using Dipeptide Buffers

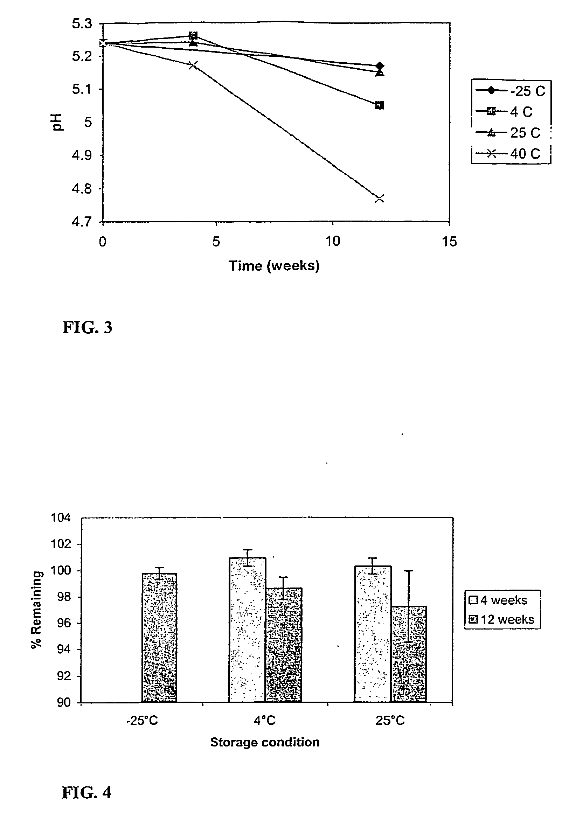

An accelerated formulation stability study was conducted to assess the buffering capabilities of dipeptides during storage. Hydrogels were prepared containing 2.37% ROH-4746 in the storage composition using the method describe above, and buffered with His-Glu (pI = 5.2, 25 mM) having an initial pH of 5.24. Furthermore, the stability of a ROH-4746 under storage in a multipeptide buffer was also assessed. FIG. 3 is a graph that shows the shift in pH over a 12-week period in varied storage conditions. A maximum pH shift was seen to be a decrease of about 0.7 unit in hydrogels stored at 40° C. However, in storage conditions of 25° C and below, pH shift was limited to about 0.4 pH units or less. In all cases, the multipeptide was shown to be sufficient in maintaining pH during storage. This study showed the utility of multipeptide buffers within the formulation. FIG. 4 summarizes the recovery of the drug in the His-Glu buffered hydrogels of FIG.3. In all cases under normal storage conditions at or below normal ambient room temperature the recovery was acceptable after 12 weeks of storage (normal ambient temperature can be considered to be about 27°C). For example, when stored at temperatures of about -25°C to 25°C, the loss was about 10% or less.

### EXAMPLE 4. Preparation of Anionic Drug Formulations for Electrotransport

The pH of an anionic drug is adjusted with a polymeric cation exchange material and is then buffered with a polymeric ion exchange material according to the following procedure.

The drug ceftriaxone (supplied as the disodium salt) has the following three pKa's: 3 (carboxylic), 3.2 (amine), and 4.1 (enolic OH), which act as bases in solution. The natural pH of the drug in water is higher than neutral, which is higher than the highest pKa of the drug. Using the Henderson-Hasselbach equation, the theoretical final pH of this system can be calculated.

A polymeric cation exchange material is added to a solution of ceftriaxone to adjust the pH of the solution to a value between that of the second and third pKa's of the drug, to be at or near the pI of the multipeptide-containing peptidic buffer (e.g., Gly-Asp with pI = 3.6). An appropriate peptidic buffer is then used to buffer the solution to maintain the pH during storage and/or operation of an electrotransport device (e.g., Gly-Asp pI =3.6 at 10-70 mM). The concentration of ceftriazone can be selected from about 1 to 30wt%, preferably about 2 to 10wt% on an aqueous basis, in the ion exchanged drug solution, as well as in the resulting hydrogel on an aqueous basis.

Based on the present invention disclosure, it is evident to one skilled in the art that such a buffered anionic drug composition will have improved pH stability than the anionic drug without buffering and have less competing anions than the drug buffered with acids such as inorganic HCl, and the like, and other traditional nonpeptidic buffers.

As a result of using a first ion exchanger to adjust the drug solution, removing the first ion exchanger from the drug solution and buffering the resulting drug solution with peptidic buffering, a drug solution with storage and electrotransport pH stability with minimal competing ions can be obtained. For example, for ceftriaxone, the resulting buffered drug solution can have a ceftriaxone concentration of about 1-30wt%, preferably 2-10wt%, with a pH from about 3.5 to 4.0, preferably about 3.4 to 3.9, on the steep slope of the titration curve of the drug, with a strong acid anionic ion concentration of less than about 75mM, preferably less than about 20mM, and more preferably less than about 10mM. The concentration of a multipeptide buffer is generally from about 10 mM to 250mM, preferably from about 15 mM to 100mM, more preferably from about 25mM to 70mM.

A similar approach is used for the drug Cefodizime disodium salt with pKa values of 2.85, 3.37, and 4.18. As a result of using a first ion exchange to adjust the drug solution, removing the first ion exchange from the drug solution and buffering the resulting drug solution with a peptidic buffering, a drug solution with storage and electrotransport pH stability with minimal competing ions can be obtained. For example, for Cefodizime disodium salt, the resulting buffered drug solution can have a Cefodizime concentration of about 1 to 30wt%, preferably about 2 to 10wt%, with a pH of from about 3.4 to 4.1, preferably about 3.5 to 3.9, on the steep slope of the titration curve of the drug, with a strong acid anionic ion concentration of less than about 75mM, preferably less than about 40mM, and more preferably about 20mM. The concentration of a multipeptide buffer is generally from about 10 mM to 250mM, preferably from about 20mM to 100mM, more preferably from about 25 mM to 70mM. The concentration of Cefodizime disodium salt can be selected from about 1 to 30wt%, preferably about 2 to 10wt% on an aqueous basis, in the ion exchanged drug solution, as well as in the resulting hydrogel on an aqueous basis.

## Claims

1. A method for preparing a composition for use in an electrotransport delivery system comprising:
providing a drug solution comprising drug ions and associated counterions of a drug, the drug solution having a pH if pure;
selecting a pH range for acceptable composition stability and effective transdermal delivery, the selected pH range at least overlaps a steep slope of a titration curve of the drug solution, the pH of the drug solution if pure being outside the selected pH range;
selecting a multipeptide having an isoelectric point (pI) that falls on the steep slope of a titration curve of the drug solution;
adjusting the pH of the drug solution to about the pI of the multipeptide by contacting the drug solution with an ion exchange material;
separating the ion exchange material from the pH-adjusted drug solution; and
buffering the pH-adjusted drug solution with the multipeptide to the selected pH range.

2. The method of claim 1 wherein the drug has at least a first pKa and the multipeptide is selected such that the first pKa is between the pI of multipeptide and the pH of the drug solution if pure and such that the pH of the drug solution is adjusted across the first pKa with the ion exchange material.

3. The method of any of the preceding claims further comprising determining a stability pH range in which the drug is stable, and selecting the pH range to be within the stability pH range.

4. The method of any of the preceding claims comprising adjusting the pH of the drug solution to within 1 pH unit of the pI of the multipeptide by contacting the drug solution with an ion exchange material.

5. The method of any of the preceding claims further comprising selecting the pH range to fall on the steep slope of the titration curve, the steep slope being proximate and towards the neutral side the first pKa.

6. The method of any of the preceding claims wherein the steep slope is ranged at within 1.5 pH units on each side of an inflection point on a titration curve of the drug and wherein the selected pH range is less than 1 pH unit wide, and the steep slope of the titration curve encompasses the selected pH range of the drug.

7. The method of any of the preceding claims further comprising adding the pH-adjusted drug solution to a reservoir of an electrotransport delivery system.

8. The method of any of the preceding claims further comprising including the multipeptide in the composition to achieve a concentration 10mM to 250mM.

9. The method of any of the preceding claims wherein the multipeptide has a pI between 3 and 10.

10. The method of any of the preceding claims comprisng selecting a dipeptide or a tripeptide as the multipeptide and having at least one amino acid selected from the group consisting of His, Tyr, Arg, Cys, Lys, Asp, and Glu.

11. The method of any of the preceding claims comprisng selecting a dipeptide as the multipeptide, selected from the group consisting of, Asp-His, Glu-His, His-Glu, His-Asp, Glu-Arg, Glu-Lys, Arg-Glu, Lys-Glu, Arg-Asp, Lys-Glu, Arg-Asp, Lys-Asp, and His-Gly.

12. The method of any of the preceding claims wherein the drug ions are cationic, the associated counterions are anionic, and the ion exchange material is a polymeric anion exchange resin.

13. The method of any of the preceding claims wherein the pH of the drug solution is adjusted to between pH 3 and pH 9 by ion exchange.

14. The method of any of the preceding claims wherein the ion exchange material is a polymeric anion exchange membrane.

15. The method of any of the preceding claims wherein the drug is a cationic drug and is a factor Xa inhibitor.

16. The method of any of the preceding claims wherein the drug is a cationic drug and is a benzamidine derivative.

17. The method of any of the preceding claims comprising:
providing a drug solution comprising drug ions and associated counterions of benzamidine derivative (BD), BD having at least a first pKa; the first pKa being the lowest pKa of BD, the drug solution having a pH;
selecting a pH range for the composition, the pH range being higher than the first pKa of BD and within a range from 4 to 6.5 such that BD is stable and can be delivered effectively;
selecting a multipeptide having an isoelectric point (pI) that falls on a steep slope of a titration curve of BD;
adjusting the pH of the drug solution to pass across the first pKa to about the pI of the multipeptide by contacting the drug solution with an anionic ion exchange material;
separating the ion exchange material from the pH-adjusted drug solution; and
buffering the pH-adjusted drug solution with the multipeptide to a pH range from 4 to 6.5.

18. The method of any of the preceding claims comprising adjusting the pH of the drug solution by ion exchange to between a range of 0.5 pH unit on each side of the pI of the multipeptide.

19. The method of any of the preceding claims comprising using dipeptide His-Glu for buffering and wherein the pH of the drug solution is adjusted by ion exchange to between pH 5.0 and pH 5.5.

20. A benzamidine derivative (BD) composition for use in an electrotransport delivery system comprising:
a drug solution comprising drug ions and associated counterions of BD, BD having at least a first pKa; the first pKa being the lowest pKa of BD;
multipeptide buffer having an isoelectric point (pI) that proximate to and higher than the first pKa of BD;
wherein pH of the composition being in a range from pH 5.0 to pH 5.5 and wherein alkali cation is at a concentration of less than 75mM.

21. The benzamidine derivative (BD) composition of claim 20 wherein alkali cation is at a concentration of less than 40mM.

22. The benzamidine derivative (BD) composition of any of claims 20-21 wherein the composition contains substantially no ion exchange material.

23. The benzamidine derivative (BD) composition of any of claims 20-22 wherein the multiptptide buffer comprises His-Glu as buffering dipeptide and wherein the pH of the composition is in the range from pH 5.0 to pH 5.5.

24. The benzamidine derivative (BD) composition of any of claims 20-23
wherein pH of the composition being between pH 5.0 and pH 5.5; and wherein the composition contains substantially no ion exchange material and wherein alkali cation concentration is less than 75mM.

25. The benzamidine derivative (BD) composition of any of claims 20-24 wherein alkali cation concentration is less than 40mM.

26. The benzamidine derivative (BD) composition of any of claims 20-25 wherein BD is at a concentration of 30mM to 750mM.

27. The benzamidine derivative (BD) composition of any of claims 20-26 wherein the BD is ROH-4746.

28. The benzamidine derivative (BD) composition of any of claims 20-27 wherein the BD composition is in a reservoir of an electrotransport delivery system.

## Patentansprüche

1. Verfahren zur Herstellung einer Zusammensetzung zur Verwendung in einem Elektrotransportabgabesystem umfassend:
Bereitstellen einer Arzneimittellösung umfassend Arzneimittelionen und assoziierte Gegenionen eines Arzneimittels, die Arzneimittellösung hat einen pH-Wert, wenn sie rein ist;
Auswählen eines pH-Wert-Bereiches für eine akzeptable Stabilität der Zusammensetzung und wirksame transdermale Abgabe, der ausgewählte pH-Wert-Bereich überlappt wenigstens den steilen Abfall einer Titrationskurve der Arzneimittellösung, der pH-Wert der Arzneimittellösung, wenn sie rein ist, liegt außerhalb des ausgewählten pH-Wert-Bereiches;
Auswählen eines Multipeptids mit einem isoelektrischen Punkt (pl), der in den steilen Abfall der Titrationskurve der Arzneimittellösung fällt;
Einstellen des pH-Wertes der Arzneimittellösung auf etwa den pl-Wert des Multipeptids durch Inkontaktbringen mit einem Ionenaustauschmaterial;
Trennen des lonenaustauschmaterials von der pH-Wert-eingestellten Arzneimittellösung; und
Puffern der pH-Wert eingestellten Arzneimittellösung mit dem Mulitpeptid auf den ausgewählten pH-Wert-Bereich.

2. Verfahren nach Anspruch 1, bei dem das Arzneimittel wenigstens einen ersten pKa-Wert hat und das Multipeptid so ausgewählt ist, daß der erste pKa-Wert zwischen dem pl-Wert des Multipeptids und dem pH-Wert der Arzneimittellösung, wenn sie rein ist, liegt und daß der pH-Wert der Arzneimittellösung über den ersten pKa-Wert mit dem lonenaustauschmaterial eingestellt ist.

3. Verfahren nach einem der vorherigen Ansprüche des weiteren umfassend
Bestimmen eines Stabilitäts-pH-Wert-Bereiches, bei dem das Arzneimittel stabil ist und Auswahl des pH-Wert-Bereiches, um innerhalb des Stabilitäts-pH-Wert-Bereiches zu liegen.

4. Verfahren nach einem der vorherigen Ansprüche umfassend
Einstellen des pH-Wertes der Arzneimittellösung innerhalb einer 1 pH-Werteinheit des pl-Wertes des Multipeptids durch Inkontaktbringen der Arzneimittellösung mit einem lonenaustauschmaterial.

5. Verfahren nach einem der vorherigen Ansprüche des weiteren umfassend
Auswählen des pH-Wert-Bereichs, so daß dieser um in den steilen Abfall der Titrationskurve fällt, der steile Abfall ist benachbart zu und in Richtung der neutralen Seite des ersten pKa-Wertes.

6. Verfahren nach einem der vorherigen Ansprüche, bei dem der steile Abfall sich innerhalb von 1,5 pH-Werteinheiten auf jeder Seite eines Wendepunktes einer Titrationskurve des Arzneimittels erstreckt und bei dem der ausgewählte pH-Wert-Bereich weniger als 1 pH-Werteinheit breit ist und der steile Abfall der Titrationskurve den ausgewählten pH-Wert-Bereich des Arzneimittels begrenzt.

7. Verfahren nach einem der vorherigen Ansprüche des weiteren umfassend
Zugabe der pH-Wert-eingestellten Lösung zu einem Reservoir eines Elektrotransportabgabesystems.

8. Verfahren nach einem der vorherigen Ansprüche des weiteren umfassend, um einen Konzentration von 10 mM bis 250 mM, einschließlich des Multipeptids in der Zusammensetzung, zu erreichen.

9. Verfahren nach einem der vorherigen Ansprüche, bei dem das Multipeptid einen pl-Wert zwischen 3 und 10 hat.

10. Verfahren nach einem der vorherigen Ansprüche umfassend
Auswählen eines Dipeptids oder eines Tripeptids als Multipeptid und enthaltend wenigstens eine Aminosäure ausgewählt aus der Gruppe bestehend aus His, Tyr, Arg, Cys, Lys, Asp und Glu.

11. Verfahren nach einem der vorherigen Ansprüche umfassend
Auswählen eines Dipeptids als Multipeptid ausgewählt aus der Gruppe bestehend aus Asp-His, Glu-His, His-Glu, His-Asp, Glu-Arg, Glu-Lys, Arg-Glu, Lys-Glu, Arg-Asp, Lys-Glu, Arg-Asp, Lys-Asp und His-Gly.

12. Verfahren nach einem der vorherigen Ansprüche, bei dem die Arzneimittelionen kationisch, die assoziierten Gegenionen anionisch sind und das lonenaustauschmaterial ein polymeres lonenaustauschmaterial ist.

13. Verfahren nach einem der vorherigen Ansprüche, bei dem der pH-Wert der Arzneimittellösung auf einen pH-Wert zwischen pH 3 und pH 9 durch Ionenaustausch eingestellt ist.

14. Verfahren nach einem der vorherigen Ansprüche, bei dem das Ionenaustauschmaterial eine polymere Anionaustauschmembran ist.

15. Verfahren nach einem der vorherigen Ansprüche, bei dem das Arzneimittel ein kationisches Arzneimittel und ein Faktor Xa-Inhibitor ist.

16. Verfahren nach einem der vorherigen Ansprüche, bei dem das Arzneimittel ein kationisches Arzneimittel und ein Benzamidinderivat ist.

17. Verfahren nach einem der vorherigen Ansprüche umfassend
Bereitstellen einer Arzneimittellösung umfassend Arzneimittelionen und assoziierte Gegenionen eines Benzamidinderivats (BD), BD hat wenigstens einen ersten pKa-Wert; der erste pKa-Wert ist der niedrigste pKa-Wert des BD, die Arzneimittellösung hat einen pH-Wert;
Auswählen eines pH-Wert-Bereiches für die Zusammensetzung, der pH-Wert-Bereich ist höher als der erste pKa-Wert des BD und innerhalb eines Bereichs von 4 bis 6,5, so daß BD stabil ist und wirksam freigesetzt werden kann;
Auswählen eines Multipeptids mit einem isoelektrischen Punkt (pl), der in den steilen Abfall der Titrationskurve des BD fällt;
Einstellen des pH-Wertes der Arzneimittellösung, um über den ersten pKa-Wert auf etwa den pl-Wert des Multipeptids durch Inkontaktbringen der Arzneimittellösung mit einem anionischen lonenaustauschmaterial hinauszugehen;
Trennen des lonenaustauschmaterials von der pH-Wert eingestellten Arzneimittellösung; und
Puffern der pH-Wert-eingestellten Arzneimittellösung mit dem Multipeptid auf einen pH-Wert-Bereich von 4 bis 6.5.

18. Verfahren nach einem der vorherigen Ansprüche umfassend
Einstellen des pH-Wertes der Arzneimittellösung durch Ionenaustausch auf einen Bereich zwischen 0,5 pH-Werteinheiten auf jeder Seite des pl-Wertes des Multipeptids.

19. Verfahren nach einem der vorherigen Ansprüche umfassend
Verwendung des Dipeptids His-Glu zum Puffern und bei dem der pH-Wert der Arzneimittelmittellösung durch Ionenaustausch auf zwischen pH 5,0 und pH 5,5 eingestellt ist.

20. Benzamidinderivat (BD)-zusammensetzung zur Verwendung in einem Elektrotransportabgabeystem umfassend
eine Arzneimittellösung umfassend Arzneimittelionen und assoziierte Gegenionen des BD, BD hat wenigstens einen ersten pKa-Wert; der erste pKa-Wert ist der niedrigste pKa-Wert des BD;
einen Multipeptidpuffer mit einem isoelektrischen Punkt (pl), der nahe bei oder höher als der erste pKa-Wert des BD ist;
wobei der pH-Wert der Zusammensetzung im einem Bereich von pH 5,0 bis pH 5,5 liegt und wobei alkalisches Kation in einer Konzentration von weniger als 75 mM vorhanden ist.

21. Benzamidinderivat (BD)-zusammensetzung nach Anspruch 20, bei der alkalisches Kation in einer Konzentration von weniger als 40 mM vorhanden ist.

22. Benzamidinderivat (BD)-zusammensetzung nach einem der Ansprüche 20 bis 21, bei der die Zusammensetzung im wesentlichen kein Ionenaustauschmaterial enthält.

23. Benzamidinderivat (BD)-zusammensetzung nach einem der Ansprüche 20 bis 22, bei der der Multipeptidpuffer His-Glu als Pufferdipeptid umfaßt und bei dem der pH-Wert der Zusammensetzung im Bereich von pH 5,0 bis pH 5,5 liegt.

24. Benzamidinderivat (BD)-zusammensetzung nach einem der Ansprüche 20 bis 23, bei der der pH-Wert der Zusammensetzung zwischen pH 5,0 und pH 5,5 liegt und bei der die Zusammensetzung im wesentlichen kein Ionenaustauschmatrial enthält und bei der die alkalische Kationenkonzentration weniger als 75 mM ist.

25. Benzamidinderivat (BD)-zusammensetzung nach einem der Ansprüche 20 bis 24, bei der die alkalische Kationenkonzentration weniger als 40 mM beträgt:

26. Benzamidinderivat (BD)-zusammensetzung nach einem der Ansprüche 20 bis 25, bei der BD in einer Konzentration von 30 mM bis 750 mM vorhanden ist.

27. Benzamidinderivat (BD)-zusammensetzung nach einem der Ansprüche 20 bis 26, bei der BD ROH-4746 ist.

28. Benzamidinderivat (BD)-zusammensetzung nach einem der Ansprüche 20 bis 27, bei der die BD-Zusammensetzung in einem Reservoir eines Elektrotransportabgabesystem vorhanden ist.

## Revendications

1. Procédé de préparation d'une composition destinée à être utilisée dans un système d'administration par électrotransport comprenant les opérations consistant à :
- se procurer une solution de médicament comprenant des ions de médicament et des contre-ions associés d'un médicament, la solution de médicament ayant un pH si elle est pure ;
- choisir une plage de pH pour une stabilité de composition acceptable et une administration transdermique effective, la plage de pH choisie au moins chevauchant une pente forte d'une courbe de titration de la solution de médicament, le pH de la solution de médicament si elle est pure étant à l'extérieur de la plage de pH choisie ;
- choisir un multipeptide ayant un point isoélectrique (pI) qui tombe sur la pente forte d'une courbe de titration de la solution de médicament ;
- ajuster le pH de la solution de médicament à environ le pI du multipeptide par mise en contact de la solution de médicament avec une matière échangeuse d'ions ;
- séparer la matière échangeuse d'ions de la solution de médicament à pH ajusté ; et
- tamponner la solution de médicament à pH ajusté avec le multipeptide par rapport à la plage de pH sélectionnée.

2. Procédé selon la revendication 1, dans lequel le médicament a au moins un premier pKa et le multipeptide est choisi de telle sorte que le premier pKa est entre le pI du multipeptide et le pH de la solution de médicament si elle est pure et de telle sorte que le pH de la solution de médicament est ajusté à travers le premier pKa avec la matière échangeuse d'ions.

3. Procédé selon l'une quelconque des revendications précédentes, comprenant en outre la détermination d'une plage de pH de stabilité dans laquelle le médicament est stable, et le choix de la plage de pH pour qu'elle se situe à l'intérieur de la plage de pH de stabilité.

4. Procédé selon l'une quelconque des revendications précédentes, comprenant l'ajustement du pH de la solution de médicament à une valeur se situant dans 1 unité de pH du pI du multipeptide par mise en contact de la solution de médicament avec une matière échangeuse d'ions.

5. Procédé selon l'une quelconque des revendications précédentes; comprenant en outre le choix de la plage de pH pour qu'elle tombe sur la pente forte de la courbe de titration, la pente forte étant proche du et vers le côté neutre du premier pKa.

6. Procédé selon l'une quelconque des revendications précédentes, dans lequel la pente forte s'étend dans 1,5 unité de pH de chaque côté d'un point d'inflexion sur une courbe de titration du médicament, et dans lequel la plage de pH choisie est inférieure à 1 unité de pH de largeur, et la pente forte de la courbe de titration englobe la plage de pH choisie du médicament.

7. Procédé selon l'une quelconque des revendications précédentes, comprenant en outre l'addition de la solution de médicament à pH ajusté à un réservoir d'un système d'administration par électrotransport.

8. Procédé selon l'une quelconque des revendications précédentes, comprenant en outre l'inclusion du multipeptide dans la composition pour parvenir à une concentration de 10 mM à 250 mM.

9. Procédé selon l'une quelconque des revendications précédentes, dans lequel le multipeptide a un pI entre 3 et 10.

10. Procédé selon l'une quelconque des revendications précédentes, comprenant le choix d'un dipeptide ou un tripeptide en tant que multipeptide et ayant au moins un acide aminé choisi dans le groupe constitué par His, Tyr, Arg, Cys, Lys, Asp et Glu.

11. Procédé selon l'une quelconque des revendications précédentes, comprenant la sélection d'un dipeptide comme multipeptide, choisi dans le groupe constitué par Asp-His, Glu-His, His-Glu, His-Asp, Glu-Arg, Glu-Lys, Arg-Glu, Lys-Glu, Arg-Asp, Lys-Glu, Arg-Asp, Lys-Asp et His-Gly.

12. Procédé selon l'une quelconque des revendications précédentes, dans lequel les ions de médicament sont cationiques, les contre-ions associés sont anioniques, et la matière échangeuse d'ions est une résine échangeuse d'anions polymère.

13. Procédé selon l'une quelconque des revendications précédentes, dans lequel le pH de la solution de médicament est ajusté à une valeur entre pH 3 et pH 9 par échange d'ions.

14. Procédé selon l'une quelconque des revendications précédentes, dans lequel la matière échangeuse d'ions est une membrane échangeuse d'anions polymère.

15. Procédé selon l'une quelconque des revendications précédentes, dans lequel le médicament est un médicament cationique et est un inhibiteur du facteur Xa.

16. Procédé selon l'une quelconque des revendications précédentes, dans lequel le médicament est un médicament cationique et est un dérivé de benzamidine.

17. Procédé selon l'une quelconque des revendications précédentes, comprenant les opérations consistant à :
- se procurer une solution de médicament comprenant des ions de médicament et des contre-ions associés de dérivé de benzamidine (BD), le BD ayant au moins un premier pKa ; le premier pKa étant le pKa le plus faible du BD, la solution de médicament ayant un pH ;
- choisir une plage de pH pour la composition, la plage de pH étant supérieure au premier pKa du BD et à l'intérieur d'une plage de 4 à 6,5 telle que le BD est stable et peut être administré de façon efficace ;
- choisir un multipeptide ayant un point isoélectrique (pI) qui tombe sur une pente forte d'une courbe de titration du BD ;
- ajuster le pH de la solution de médicament pour passer à travers le premier pKa à environ le pI du multipeptide par mise en contact de la solution de médicament avec une matière échangeuse d'ions anionique ;
- séparer la matière échangeuse d'ions à partir de la solution de médicament à pH ajusté ; et
- tamponner la solution de médicament à pH ajusté avec le multipeptide dans une plage de pH de 4 à 6,5.

18. Procédé selon l'une quelconque des revendications précédentes, comprenant l'ajustement du pH de la solution de médicament par échange d'ions à une valeur se situant entre une plage de 0,5 unité de pH de chaque côté du pI du multipeptide.

19. Procédé selon l'une quelconque des revendications précédentes, comprenant l'utilisation du dipeptide His-Glu pour tamponner et dans lequel le pH de la solution de médicament est ajusté par échange d'ions à une valeur entre pH 5,0 et pH 5,5.

20. Composition de dérivé de benzamidine (BD) destinée à être utilisée dans un système d'administration par électrotransport comprenant :
- une solution de médicament comprenant des ions de médicament et des contre-ions associés de BD, le BD ayant au moins un premier pKa ; le premier pKa étant le pKa le plus faible du BD ;
- un tampon multipeptide ayant un point isoélectrique (pI) qui s'approche de et est supérieur au premier pKa du BD ;
- le pH de la composition se situant dans une plage de pH 5,0 et pH 5,5 et un cation alcalin étant à une concentration de moins de 75 mM.

21. Composition de dérivé de benzamidine (BD) selon la revendication 20, dans laquelle un cation alcalin est à une concentration de moins de 40 mM.

22. Composition de dérivé de benzamidine (BD) selon l'une quelconque des revendications 20-21, dans laquelle la composition ne contient pratiquement pas de matière échangeuse d'ions.

23. Composition de dérivé de benzamidine (BD) selon l'une quelconque des revendications 20-22, dans laquelle le tampon multipeptide comprend His-Glu comme dipeptide de tamponnage et dans laquelle le pH de la composition se situe dans la plage de pH 5,0 à pH 5,5.

24. Composition de dérivé de benzamidine (BD) selon l'une quelconque des revendications 20-23, dans laquelle le pH de la composition est situé entre pH 5,0 et pH 5,5 ; et dans laquelle la composition ne contient pratiquement pas de matière échangeuse d'ions et dans laquelle la concentration de cations alcalins est inférieure à 75 mM.

25. Composition de dérivé de benzamidine (BD) selon l'une quelconque des revendications 20-24, dans laquelle la concentration de cations alcalins est inférieure à 40 mM.

26. Composition de dérivé de benzamidine (BD) selon l'une quelconque des revendications 20-25, dans laquelle le BD est à une concentration de 30 mM à 750 mM.

27. Composition de dérivé de benzamidine (BD) selon l'une quelconque des revendications 20-26, dans laquelle le BD est ROH-4746.

28. Composition de dérivé de benzamidine (BD) selon l'une quelconque des revendications 20-27, dans laquelle la composition du BD est dans un réservoir d'un système d'administration par électrotransport.
